# EUROPEAN PATENT SPECIFICATION

(11) **EP 0 566 592 B1**
(45) Date of publication and mention of the grant of the patent: **14.09.1994**
(21) Application number: 92901466.0
(22) Date of filing: 16.12.1991
(51) Int. Cl.: C07D 513/04, A61K 31/54

(54) **Pyrimido-benzothiazines with lipoxygenase inhibitory action**
Pyrimido-benzothiazine mit Lipoxygenase-hemmung
Pyrimido-benzothiazines avec action inhibitrice de la lipoxygénase

(30) Priority: 07.01.1991 JP 159/91
(43) Date of publication of application: 27.10.1993
(73) Proprietor: PFIZER INC., Groton, CT 06340 (US)
(72) Inventor: SHIMADA, Kaoru, Okazaki-shi , Aichi (JP); SHISHIDO, Yuji, Chita-gun, Aichi (JP)
(74) Representative: Wood, David John
(86) International application number: US9109161
(87) International publication number: WO9212161

(56) References cited:
- DE-A- 1 695 595
- US-A- 4 845 083

## Description

### Technical Field

This invention relates to novel pyrimido-benzothiazine derivative compounds. The compounds of the present invention inhibit the action of the lipoxygenase enzyme and are useful in the treatment or alleviation of inflammatory diseases, allergy and cardiovascular diseases in mammals. This invention also relates to pharmaceutical compositions comprising such compounds.

### Background Art

Arachidonic acid is known to be the biological precursor of several groups of endogenous metabolites, prostaglandins including prostacyclins, thromboxanes and leukotrienes. The first step of arachidonic acid metabolism is the release of arachidonic acid and related unsaturated fatty acids from membrane phospholipids via the action of phospholipase. Free fatty acids are then metabolized either by cyclooxygenase to produce the prostaglandins and thromboxanes or by lipoxygenase to generate hydroperoxy fatty acids which may be further converted to the leukotrienes. Leukotrienes have been implicated in the pathophysiology of inflammatory diseases including rheumatoid arthritis, gout, asthma, ischemia reperfusion injury, psoriasis and inflammatory bowel disease. Compounds that inhibit lipoxygenase are expected to provide significant new therapy for both acute and chronic inflammatory conditions.

Recently, several review articles on lipoxygenase inhibitors have been reported. See H. Masamune and L. S. Melvin, Sr., in: Annual Reports in Medicinal Chemistry 24 (1989) pp. 71-80 (Academic); and B. J. Fitzsimmons and J. Rokach in: Leukotrienes and Lipoxygenases (1989) pp. 427-502 (Elsevier).

Offenlegungsschrift 1695595 discloses certain pyrimidobenzothiazines which are useful for blocking phosphodiesterases. Sakamuki, M. et al. and Sako, M. et al. both in 12th Symposium on Progress in Organic Reactions and Syntheses, Symposium Papers, Nov. 8-9, 1985, Nagoya, pp. 186-189 and p. 190; and Sako, M. et al., Chem. Pharm. Bull., 32, pp. 2474-2476 (1984), disclose the synthesis of certain pyrimido-benzothiazines.

Certain derivatives of phenothiazine of the general formula 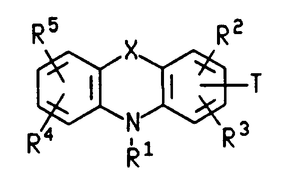
wherein X is, inter alia, S, SO or SO₂; R¹ is, inter alia, H or (C₁-C₆)alkyl; R², R³, R⁴ and R⁵ are inter alia, independently H, (C₁-C₆)alkyl, (C₂-C₆)alkenyl or -(CH₂)ₙM where n is 0-6 and M is halogen or various groups and T is H or OR¹⁵ where R¹⁵ is H or various groups are disclosed in EP 138481, published April 24, 1985 and corresponding to JP 60155165, as leukotriene biosynthesis inhibitors useful in treating allergic conditions, asthma, cardiovascular disorders, inflammation and certain skin diseases.

### Disclosure of the Invention

The present invention provides pyrimido-benzothiazine derivative compounds of the following formulae: 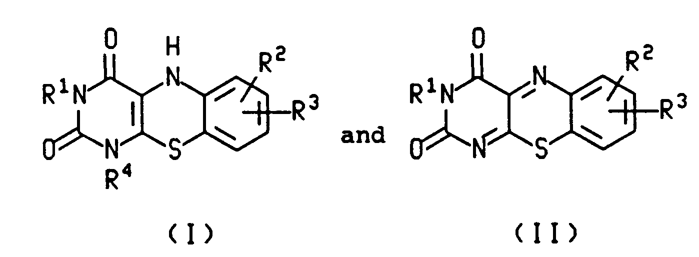
and the pharmaceutically-acceptable salts thereof, wherein R¹ is hydrogen or (C₁-C₆)alkyl; R² is hydrogen, halogen, (C₁-C₆)alkyl, (C₁-C₆)alkoxy or (C₁-C₆)halosubstituted alkyl; R³ is hydrogen or (C₁-C₆)alkyl; R⁴ is hydrogen, (C₁-C₆)alkyl, (C₃-C₈)cycloalkyl, (C₁-C₆)alkoxy-(C₁-C₆)alkyl or aryl-(C₁-C₆)alkyl; provided that in formula (I), R¹, R², R³ and R⁴ are not simultaneously hydrogen, R², R³ and R⁴ are not simultaneously hydrogen when R¹ is methyl and R² and R³ are not simultaneously hydrogen when R¹ and R⁴ are respectively methyl; provided further that in formula (II), R¹, R² and R³ are not simultaneously hydrogen and R² and R³ are not simultaneously hydrogen when R¹ is methyl; and provided further still that the groups R² and R³ may be attached to any available position on the ring in formula (I) or (II).

A preferred group of compounds comprises compounds of formula (I) wherein R¹ is (C₁-C₆)alkyl. Also preferred are compounds of formula (I) wherein R¹ is methyl and R² is halogen, (C₁-C₆)alkyl or (C₁-C₆)alkoxy. Another preferred group of compounds comprises compounds of formula (II) wherein R¹ is (C₁-C₆)alkyl. Also preferred are compounds of formula (II) wherein R¹ is methyl and R² is halogen, (C₁-C₆)alkyl, (C₁-C₆)alkoxy or (C₁-C₆)halosubstituted alkyl.

In formulae (I) and (II), above, and throughout this specification and the appendant claims,
the term "halogen" is used to mean radicals derived from the elements fluorine, chlorine, bromine and iodine;
the term "alkyl" is used to mean straight or branched chain radicals, including, but not limited to, methyl, ethyl, n-propyl, isopropyl, butyl and the like;
the term "alkoxy" is used to mean -OR₅ wherein R₅ is an alkyl radical, including, but not limited to, methoxy, ethoxy, propoxy, isopropoxy, butoxy and the like;
the term "halosubstituted alkyl" refers to an alkyl radical as described above substituted with one or more halogens, including, but not limited to chloromethyl, trifluoromethyl, 2,2,2-trichloroethyl and the like;
the term "alkoxyalkyl" is used to mean -R₆OR₇ wherein R₆ and R₇ are respectively alkyl radicals, including, but not limited to methoxymethyl, methoxyethyl, ethoxymethyl, ethoxyethyl and the like;
the term "arylalkyl" means an aryl group appended to an alkyl radical including, but not limited to, phenylmethyl-(benzyl), phenylethyl, 2-phenylethyl, phenylpropyl and 2-pyridylmethyl, and the like;
the term "cycloalkyl" is used to mean carbocyclic radicals, including, but not limited to, cyclopropyl, cyclobutyl, cyclopentyl, cyclohexyl, and the like.

Certain of the compounds of the above formula may form acid salts. The pharmaceutically-acceptable acid salts are those formed from acids which yield non-toxic acid salts, for example, hydrochloride, hydrobromide, sulfate or bisulfate, phosphate or acid phosphate, acetate, citrate, fumarate, gluconate, lactate, maleate, succinate, tartrate, methanesulfonate, benzenesulfonate, toluenesulfonate, and formate salts.

This invention includes pharmaceutical compositions for treatment of inflammatory diseases, allergy and cardiovascular diseases in a mammal which comprises a pharmaceutically-acceptable carrier or diluent and a compound of the above formula (I) or (II) or a pharmaceutically-acceptable salt thereof.

This invention further includes the uses of compounds of formula (I) and (II) for preparing medicament useful in the treatment of inflammatory diseases, allergy and cardiovascular diseases.

### Detailed Description

The compounds of formulae (I) and (II), above, can be prepared by any of a number of synthetic methods. A preferred method is shown in Scheme A and described below wherein R¹, R², R³ and R⁴ are as described above. 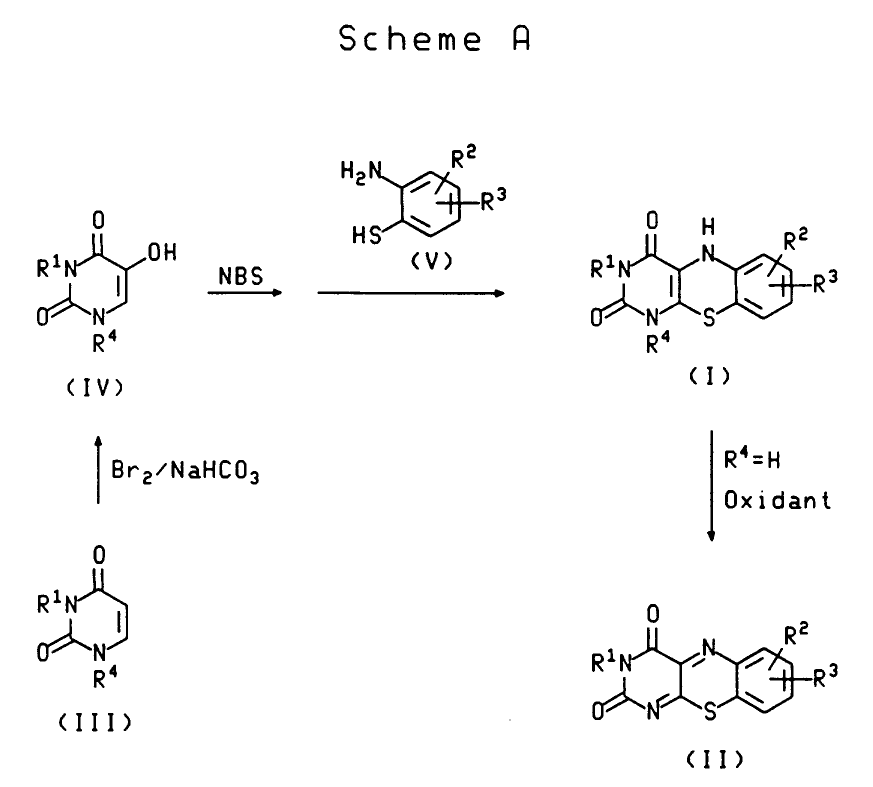
According to the synthetic method shown in Scheme A, above, bromine is added dropwise to an ice-cooled solution or suspension of a pyrimidine-2,4(1H,3H)-dione derivative of formula (III) in water until the solution or suspension is colored pale yellow. The pyrimidine-2,4(1H,3H)-dione derivatives of formula (III) are prepared, for example, by the methods described in Heterocyclic Compounds 16, The pyrimidine supplement II, D. J. Brown, An Interscience Publication, John Willy & Sons, New York, 1985, Table LVIII, page 728. To the resulting pale yellow solution or suspension is added carefully an ice cold saturated aqueous NaHCO₃ solution. The resulting reaction mixture is refluxed, the solvent is removed and the residue is recrystallized from an appropriate solvent or chromatographed over silica-gel to give a 5-hydroxypyrimidine-2,4(1H,3H)-dione derivative of formula (IV).

Then, to a solution or suspension of a 5-hydroxypyrimidine-2,4(1H,3H)-dione derivative of formula (IV) in a reaction inert solvent is added N-bromosuccinimide portionwise at room temperature. A preferred solvent is ethanol. The reaction mixture is stirred until the derivative of formula (IV) disappears. Then, a 2-aminobenzenethiol derivative of formula (V) is added and the mixture is heated at reflux. Certain 2-aminobenzenethiol derivatives of formula (V) are commercially available and, further, the derivatives of formula (V) can be prepared from the corresponding 2-aminobenzothiazoles by hydrolysis as described by Mital, R. L., et al., J. Chem. Soc. (c), 2148 (1969). When a precipitate results after reflux of the reaction mixture, the precipitate is collected to give a 1,5-dihydro-2H-pyrimido[4,5-b][1,4]benzothiazine-2,4(3H)-dione derivative of formula (I). When no precipitate is obtained after reflux, the derivative of formula (I) is obtained by chromatographic separation on silica gel. The foregoing method for reacting derivatives of formula (IV) with derivatives of formula (V) to yield derivatives of formula (I) is analogous to the method described by Sako, M., et al., Chem. Pharm. Bull., 32, 2474 (1984).

When derivative compounds of formula (II) are desired, a solution or suspension of a 1,5-dihydro-2H-pyrimido[4,5-b][1,4]benzothiazine-2,4(3H)-dione derivative of formula (I) wherein R⁴ is hydrogen in acetonitrile is prepared. To that solution or suspension at room temperature is added, dropwise, an oxidant such as diethylazodicarboxylate, 1,4-benzoquinone or 2,3-dichloro-5,6-dicyano-1,4-benzoquinone. Alternatively, oxygen can be used as the oxidant. A preferred oxidant is diethylazodicarboxylate. The reaction mixture is stirred. When a precipitate results, the precipitate is collected and washed with diethylether to give a 2H-pyrimido[4,5-b][1,4]benzothiazine-2,4(3H)-dione derivative of formula (II). When no precipitate is obtained after oxidation, the solvent is removed from the reaction mixture, and the residue is triturated with diethylether and filtered to give a compound of formula (II).

The pharmaceutically-acceptable salts of the compounds of the present invention are readily prepared by contacting said compounds with a stoichiometric amount of, in the case of non-toxic cation, an appropriate metal hydroxide or alkoxide or amine in either aqueous solution or a suitable organic solvent; or, in the case of a non-toxic acid salt, an appropriate mineral or organic acid in either aqueous solution or a suitable organic solvent. The respective salt may then be obtained by precipitation or by evaporation of the solvent.

The compounds of this invention inhibit the activity of the lipoxygenase enzyme. This inhibition has been demonstrated by an assay using rat peritoneal cavity resident cells which determines the effect of said compounds on the metabolism of arachidonic acid as described in Jap. J. Inflammation 7:145-150, 1987, "Synthesis of leukotrienes by peritoneal macrophages".

The ability of the compounds of the present invention to inhibit the lipoxygenase enzyme makes them useful for controlling the symptoms induced by the endogenous metabolites arising from arachidonic acid in a mammalian subject. The compounds therefore are valuable in the prevention and treatment of disease states in which the accumulation of arachidonic acid metabolites are the causative factor, e.g., allergic bronchial asthma, skin disorders, rheumatoid arthritis, osteoarthritis and thrombosis.

Thus, the compounds of formula (I) and (II), and their pharmaceutically-acceptable salts are of particular use in the treatment or alleviation of inflammatory diseases, allergy and cardiovascular diseases in a human subject as well as in the inhibition of the lipoxygenase enzyme.

For treatment of the various conditions described above, the compounds of this invention and their pharmaceutically-acceptable salts can be administered to a human subject either alone, or, preferably, in combination with pharmaceutically-acceptable carriers or diluents in a pharmaceutical composition, according to standard pharmaceutical practice. A compound of this invention can be administered by a variety of conventional routes of administration including oral and parenteral administration and by inhalation. When the compounds are administered orally, the dose range will be from about 0.1 to 20 mg/kg body weight of the subject to be treated per day, preferably from about 0.1 to 1.0 mg/kg per day in single or divided doses. If parenteral administration is desired, then an effective dose will be from 0.1 to 1.0 mg/kg body weight of the subject to be treated per day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of the invention and their pharmaceutically-acceptable salts can be administered, for example, in the form of tablets, powders, lozenges, syrups or capsules, or as an aqueous solution or suspension. In the case of tablets for oral use, carriers which are commonly used include lactose and corn starch. Further, lubricating agents, such as magnesium stearate, are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added. For intramuscular, intraperitoneal, subcutaneous and intravenous use, a sterile solution of the active ingredient is prepared, and the pH of the solutions should be suitably adjusted and buffered. For intravenous use, the total concentration of solutes should be controlled to make the preparation isotonic.

The present invention is illustrated by the following examples. However, it should be understood that the invention is not limited to the specific details of these examples. Proton nuclear magnetic resonance spectra (NMR) were measured at 270 MHz unless otherwise indicated and peak positions are expressed in parts per million (ppm) downfield from tetramethylsilane. The peak shapes are denoted as follows: s, singlet; d, doublet; t, triplet; q, quartet; m, multiplet; br, broad.

### EXAMPLE 1

### 3-Propyl-2H-pyrimido[4,5-b][1,4]benzothiazine-2,4(3H)-dione

### A. 5-Hydroxy-3-propylpyrimidine-2,4(1H,3H)-dione

To an ice-cooled solution of 3-propylpyrimidine-2,4(1H,3H)-dione (3.00 g) in H₂O (100 ml) was added dropwise bromine (1.56 g) until the solution was colored pale yellow. An ice cold saturated NaHCO₃ solution was added carefully to the reaction mixture. The alkalized solution was refluxed for 12 hours. After removal of the solvent, the residue was recrystallized from ethanol to give 5-hydroxy-3-propylpyrimidine-2,4(1H,3H)-dione (2.32 g, 70% yield).

### B. 1,5-Dihydro-3-propyl-2H-pyrimido[4,5-b][1,4]benzothiazine-2,4(3H)-dione

To a solution of the 5-hydroxy-3-propylpyrimidine-2,4(1H,3H)-dione (0.85 g) in ethanol (40 ml), N-bromosuccinimide (0.98 g) was added in portions at room temperature. The mixture was stirred for 30 min. 2-Aminobenzenethiol (0.94 g) was added and the mixture was heated at reflux for one hour. After cooling, the precipitate was collected to give 1,5-dihydro-3-propyl-2H-pyrimido[4,5-b][1,4]benzothiazine-2,4(3H)-dione (1.20 g, 87% yield).

### C. 3-Propyl-2H-pyrimido[4,5-b][1,4]benzothiazine-2,4(3H)-dione

To a solution of the 1,5-dihydro-3-propyl-2H-pyrimido-[4,5-b][1,4]benzothiazine-2,4(3H)-dione (0.55 g) in acetonitrile (40 ml) was added dropwise diethylazodicarboxylate (0.52 g) at room temperature. The mixture was stirred for two hours. After the solvent was removed, the residue was triturated with diethylether and filtered to give 3-propyl-2H-pyrimido[4,5-b][1,4]benzothiazine-2,4(3H)-dione (0.43 g, 78% yield).
m.p.: 258°C
IR:(KBr)cm⁻¹: 2950, 1650, 1520, 1430
NMR: (DMSO-d₆) δ: 8.16 (1H, dd, 1.5Hz, 8Hz), 8.00 (1H, dd, 1.5Hz, 8Hz), 7.82 (1H, dt, 1.5Hz, 8Hz), 7.76 (1H, dt, 1.5Hz, 8Hz), 3.81 (2H, t, 7.5 Hz), 1.58 (2H, dt, 7.3Hz, 7.5Hz), 0.90 (3H, t, 7.5Hz).

### EXAMPLE 2

### 6-Chloro-1,5-dihydro-3-methyl-2H-pyrimido-[4,5-b][1,4]benzothiazine-2,4(3H)-dione

Employing the procedure of Example 1, parts A and B with 3-methylpyrimidine-2,4(1H,3H)-dione and 2-amino-3-chlorobenzenethiol yielded the title compound.
m.p.: 268-270°C (dec.)
IR:(KBr)cm⁻¹: 3360, 1697, 1638, 1463, 1307, 750
NMR: (DMSO-d₆) δ: 3.15 (3H,s), 6.34 (1H,s), 6.85 (1H, dd, 7.7Hz, 8.1Hz), 7.08 (1H, d, 7.7Hz), 7.26 (1H, d, 8.1Hz), 11.61(1H,s).

### EXAMPLE 3

### 1,5-Dihydro-3,8-dimethyl-2H-pyrimido[4,5-b]-[1,4]benzothiazine-2,4(3H)-dione

Employing the procedure of Example 1, parts A and B with 3-methylpyrimidine-2,4(1H,3H)-dione and 2-amino-5-methylbenzenethiol yielded the title compound.
m.p.: 285°C (dec.)
IR:(KBr)cm⁻¹: 3320, 2990, 1700, 1620, 1600, 1500, 1310, 815
NMR: (DMSO-d₆) δ: 11.30 (1H, s), 7.40 (1H, s), 6.90 (1H, d, 8Hz) 6.78 (1H, d, 8Hz), 3.14 (3H, s), 2.11 (3H, s).

### EXAMPLE 4

### 1,5-Dihydro-8-methoxy-3-methyl-2H-pyrimido-[4,5-b][1,4]benzothiazine-2,4(3H)-dione

Employing the procedure of Example 1, parts A and B, with 3-methylpyrimidine-2,4(1H,3H)-dione and 2-amino-5-methoxybenzenethiol yielded the title compound.
m.p.: 261.4°C
IR:(KBr)cm⁻¹: 3325, 1700, 1617, 1498, 1305, 1227, 1034, 818, 750
NMR. (DMSO-d₆) δ: 3.14 (3H, s), 3.64 (3H, s), 6.60-7.50 (4H, m), 11.31 (1H, s).

### EXAMPLE 5

### 8-Ethoxy-1,5-dihydro-3-methyl-2H-pyrimido[4,5-b]-[1,4]benzothiazine-2,4(3H)-dione

Employing the procedure of Example 1, parts A and B, with 3-methylpyrimidine-2,4(1H,3H)-dione and 2-amino-5-ethoxybenzenethiol yielded the title compound.
m.p.: 261°C (dec.)
IR:(KBr)δ: 3300, 3200, 1715, 1600, 1495, 1470
NMR: (DMSO-d₆) δ: 11.30 (1H, s), 7.29 (1H, s), 6.96 (1H, m), 6.60 (1H, m), 3.89 (2H, q, 7Hz), 3.14 (3H, s), 1.25 (3H, t, 7Hz)

### EXAMPLE 6

### 8-Fluoro-1,5-dihydro-3-methyl-2H-pyrimido[4,5-b]-[1,4]benzothiazine-2,4(3H)-dione

Employing the procedure of Example 1, parts A and B, with 3-methylpyrimidine-2,4(1H,3H)-dione and 2-amino-5-fluorobenzenethiol yielded the title compound.
m.p.: 280°C (dec.)
IR:(KBr)cm⁻¹: 3280, 3080, 1717, 1694, 1604, 1496, 1472, 1307, 1250, 1198, 897, 743
NMR: (DMSO-d₆)δ: 3.14 (3H, s), 6.86 (1H, td, 8.6, 2.6Hz), 6.97 (1H, dd, 8.6, 2.6Hz), 7.03 (1H, dd, 8.6, 5.1Hz), 7.60 (1H, s), 11.37 (1H, s).

### EXAMPLE 7

### 7-Trifluoromethyl-3-methyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-4-trifluoromethylbenzenethiol yielded the title compound.
m.p.: 238°C
IR:(KBr)cm⁻¹: 1730, 1670, 1585, 1555, 1535, 1340, 1310, 1290, 1210, 1175, 1120, 945
NMR: (DMSO-d₆) δ: 8.48 (1H, s), 8.25 (1H, m), 8.16 (1H, m), 3.27 (3H, s).

### EXAMPLE 8

### 3,8-Dimethyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-5-methoxybenzenethiol yielded the title compound.
m.p.: 250°C (dec.)
IR:(KBr)cm⁻¹: 1700, 1665, 1515, 1440, 1115
NMR: (DMSO-d₆) δ: 8.07 (1H, d, 8Hz), 7.83 (1H, s), 7.59 (1H, d, 8Hz), 3.25 (3H, s), 2.07 (3H, s).

### EXAMPLE 9

### 8-Ethoxy-3-methyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-5-ethoxybenzenethiol yielded the title compound.
m.p.: 280°C (dec.)
IR:(KBr)cm⁻¹: 1700, 1660, 1520, 1235
NMR: (DMSO-d₆) δ: 8.11 (1H, d, 8Hz), 7.65 (1H, s), 7.34 (1H, d, 8Hz), 4.26 (2H, q, 7Hz), 3.24 (3H, s), 1.40 (3H, t, 7Hz).

### EXAMPLE 10

### 8-Chloro-1,5-dihydro-3-methyl-2H-pyrimido-[4,5-b][1,4]benzothiazine-2,4(3H)-dione

Employing the procedure of Example 1, parts A and B, with 3-methylpyrimidine-2,4(1H,3H)-dione and 2-amino-5-chlorobenzenethiol yielded the title compound.
m.p.: 288.3°C
IR:(KBr)cm⁻¹: 3275, 3200, 3104, 1718, 1694, 1600, 1471, 1305, 813, 742
NMR: (DMSO-d₆) δ: 3.13 (3H, s), 6.97-7.05 (2H, m), 7.08 (1H, d, 2.2Hz), 7.77 (1H, s), 11.38 (1H, s).

### EXAMPLE 11

### 1,5-Dihydro-3,7,8-trimethyl-2H-pyrimido[4,5-b]-[1,4]benzothiazine-2,4(3H)-dione

Employing the procedure of Example 1, parts A and B, with 3-methylpyrimidine-2,4(1H,3H)-dione and 2-amino-4,5-dimethylbenzenethiol yielded the title compound.
m.p.: >300°C
IR:(KBr)cm⁻¹: 3340, 1698, 1621, 1300, 903, 753
NMR: (DMSO-d₆) δ: 2.04 (6H, s), 3.13 (3H, s), 6.71 (1H, s), 6.81 (1H, s), 7.26 (1H, s), 11.29 (1H, s).

### EXAMPLE 12

### 1,5-Dihydro-1-methoxyethyl-3-methyl-2H-pyrimido-[4,5-b][1,4]benzothiazine-2,4(3H)-dione

Employing the procedure of Example 1, parts A and B, with 1-methoxyethyl-3-methylpyrimidine-2,4(1H,3H)-dione and 2-aminobenzenethiol yielded the title compound.
m.p.: 130°C
IR:(KBr)cm⁻¹: 3330, 1685, 1620, 1585, 1445, 1120, 750
NMR: (DMSO-d₆) δ: 7.81 (1H, s), 7.03-7.14 (3H, m), 6.82 (1H, dt, 1.5Hz, 7.3Hz), 4.05 (2H, t, 5.5Hz), 3.54 (2H, t, 5.5Hz), 3.27 (3H, s), 3.21 (3H, s).

### EXAMPLE 13

### 1,5-Dihydro-3-propyl-2H-pyrimido[4,5-b]-[1,4]benzothiazine-2,4(3H)-dione

Employing the procedure of Example 1, parts A and B, with 3-n-propylpyrimidine-2,4(1H,3H)-dione and 2-aminobenzenethiol yielded the title compound.
m.p.: 287°C
IR:(KBr)cm⁻¹: 3255, 1695, 1605, 1575, 1305, 1260, 930
NMR: (CDCl₃) δ: 11.33 (1H, s), 7.54 (1H, s), 6.93-7.00 (3H, m), 6.76 (1H, m), 3.73 (2H, t, 7.3Hz), 1.53 (2H, m), 0.84 (3H, t, 7.3Hz).

### EXAMPLE 14

### 3-Butyl-1,5-dihydro-2H-pyrimido[4,5-b]-[1,4]benzothiazine-2,4(3H)-dione

Employing the procedure of Example 1, parts A and B, with 3-butylpyrimidine-2,4(1H,3H)-dione and 2-aminobenzene-thiol yielded the title compound.
m.p.: 277-278°C (dec.)
IR:(KBr)cm⁻¹: 3300, 3100, 2960, 1712, 1603, 1465, 1306, 1260, 753, 740
NMR: (DMSO-d₆) δ: 0.89 (3H, t, 7.3Hz), 1.27 (2H, qt, 7.3, 7.2Hz), 1.50 (2H, tt, 7.3, 7.2Hz), 3.77 (2H, t, 7.3Hz), 6.72-6.77 (1H, m), 6.94 (1H, d, 7.3Hz), 6.97-7.00 (2H, m), 7.54 (1H, s), 11.33 (1H, s).

### EXAMPLE 15

### 1,5-Dihydro-1-isopropyl-3-methyl-2H-pyrimido-[4,5-b][1,4]benzothiazine-2,4(3H)-dione

Employing the procedure of Example 1, parts A and B, with 1-isopropyl-3-methylpyrimidine-2,4(1H,3H)-dione and 2-aminobenzenethiol yielded the title compound.
m.p.: 167°C
IR:(KBr)cm⁻¹: 3330, 3280, 1690, 1630, 1455, 745
NMR: (CDCl₃) δ: 7.09 (1H, dt, 1.5Hz, 8Hz), 7.01 (1H, d, 8Hz), 6.87 (1H, dt, 1.5Hz, 8Hz), 6.72 (1H, dd, 1.5Hz, 8Hz), 6.26 (1H, s), 4.85 (1H, sev, 7Hz), 3.34 (3H, s), 1.58 (6H, d, 7Hz).

### EXAMPLE 16

### 7-Trifluoromethyl-1,5-dihydro-3-methyl-2H-pyrimido-[4,5-b][1,4]benzothiazine-2,4(3H)-dione

Employing the procedure of Example 1, parts A and B, with 3-methylpyrimidine-2,4(1H,3H)-dione and 2-amino-4-trifluoromethylbenzenethiol yielded the title compound.
m.p.: >300°C
IR:(KBr)cm⁻¹: 3350, 1710, 1610, 1330, 1120, 940, 750
NMR: (DMSO-d₆) δ: 11.45 (1H, s), 7.97 (1H, d, 1.8Hz), 7.14 (1H, d, 8Hz), 7.01 (1H, dd, 8Hz, 1.8Hz).

### EXAMPLE 17

### 7-Trifluoromethyl-1,5-dihydro-3-propyl-2H-pyrimido[4,5-b][1,4]benzothiazine-2,4(3H)-dione · Hydrobromide

Employing the procedure of Example 1, parts A and B, with 3-n-propylpyrimidine-2,4(1H,3H)-dione and 2-amino-4-trifluoromethylbenzenethiol yielded the title compound.
m.p.: >300°C
IR:(KBr)cm⁻¹: 3280, 1710, 1610, 1460, 1330, 1120
NMR: (DMSO-d₆) δ: 11.43 (1H, s), 7.96 (1H, s), 7.34 (1H, s), 7.17 (1H, d, 8Hz), 7.03 (1H, d, 8Hz), 3.73 (2H, t, 7.3Hz), 1.53 (2H, m), 0.85 (3H, t, 7.3Hz).

### EXAMPLE 18

### 3-Butyl-7-trifluoromethyl-1,5-dihydro-2H-pyrimido-[4,5-b][1,4]benzothiazine-2,4(3H)-dione

Employing the procedure of Example 1, parts A and B, with 3-butylpyrimidine-2,4(1H,3H)-dione and 2-amino-4-trifluoromethylbenzenethiol yielded the title compound.
m.p.: >300°C
IR:(KBr)cm⁻¹: 3250, 2975, 1710, 1610, 1460, 1330, 950, 750
NMR: (DMSO-d₆) δ: 0.89 (2H, t, 7.3Hz), 1.27 (2H, qt, 7.3, 7.3Hz), 1.50 (2H, tt, 7.3, 7.1Hz), 3.76 (2H, t, 7.1Hz), 7.02 (1H, dd, 8.1, 1.5Hz), 7.15 (1H, d, 8.1Hz), 7.34 (1H, d, 1.5Hz), 7.95 (1H, s), 11.4 (1H, s).

### EXAMPLE 19

### 1-Cyclopentyl-1,5-dihydro-3-methyl-2H-pyrimido-[4,5-b][1,4]benzothiazine-2,4(3H)-dione

Employing the procedure of Example 1, parts A and B, with 1-cyclopentyl-3-methylpyrimidine-2,4(1H,3H)-dione and 2-aminobenzenethiol yielded the title compound.
m.p.: 166°C
IR:(KBr)cm⁻¹: 3400, 1690, 1630, 1480, 1340, 1300, 1180
NMR: (CDCl₃) δ: 7.08 (1H, dt, 1.5Hz, 7.5Hz), 7.01 (1H, dd, 1.5Hz, 7.5Hz), 6.87 (1H, dt, 1.5Hz, 7.5Hz), 6.71 (1H, dd, 1.5Hz, 7.5Hz), 6.25 (1H, s), 4.75 (1H, s), 3.35 (3H, s), 2.21 (2H, m), 1.95 (4H, m), 1.60 (2H, m).

### EXAMPLE 20

### 1-Benzyl-1,5-dihydro-3-methyl-2H-pyrimido[4,5-b]-[1,4]benzothiazine-2,4(3H)-dione

Employing the procedure of Example 1, parts A and B, with 1-benzyl-3-methylpyrimidine-2,4(1H,3H)-dione and 2-aminobenzenethiol yielded the title compound.
m.p.: 193°C
IR:(KBr)cm⁻¹: 3320, 1685, 1625, 1580, 1460, 740
NMR: (DMSO-d₆) δ: 7.88 (1H, s), 7.28-7.37 (5H, m), 7.08 (1H, m), 7.06 (1H, m), 6.94 (1H, m), 6.81 (1H, m), 5.14 (2H, s), 3.25 (3H, s).

### EXAMPLE 21

### 6-Chloro-3-methyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-3-chlorobenzenethiol yielded the title compound.
m.p.: 271-273°C (dec.)
IR:(KBr)cm⁻¹: 1716, 1662, 1527, 1130, 787, 742
NMR: (DMSO-d₆) δ: 3.16 (3H, s), 7.60-7.78 (3H, m).

### EXAMPLE 22

### 8-Fluoro-3-methyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-5-fluorobenzenethiol yielded the title compound.
m.p.: >300°C
IR:(KBr)cm⁻¹: 1571, 1518, 1293, 1278, 1230, 1216, 1138, 1118
NMR: (DMSO-d₆) δ: 3.25 (3H, s), 7.63 (1H, td, 8.8, 2.9Hz), 8.05 (1H, dd, 8.8, 2.9Hz), 8.26 (1H, dd, 5.7, 8.8Hz).

### EXAMPLE 23

### 8-Methoxy-3-methyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-5-methoxybenzenethiol yielded the title compound.
m.p.: 258.5°C
IR:(KBr)cm⁻¹: 1700, 1654, 1511, 1339, 1233, 1118
NMR: (DMSO-d₆) δ: 3.24 (3H, s), 3.97 (3H, s), 7.35 (1H, dd, 8.8, 2.9Hz), 7.67 (1H, d, 2.9Hz), 8.11 (1H, d, 8.8Hz).

### EXAMPLE 24

### 3,7,8-Trimethyl-2H-pyrimido[4,5-b]-[1,4]benzothiazine-2,4(3H)-dione

Employing the procedure of Example 1, parts A, B and C, with 3-methylpyrimidine-2,4(1H,3H)-dione and 2-amino-4,5-dimethylbenzenethiol yielded the title compound.
m.p.: >300°C
IR:(KBr)cm⁻¹: 1703, 1680, 1522, 1237, 1128
NMR: (DMSO-d₆) δ: 2.40 (3H, s), 2.42 (3H, s), 3.25 (3H, s), 7.81 (1H, s), 8.01 (1H, s).

### EXAMPLE 25

### 3-Butyl-2H-pyrimido[4,5-b][1,4]benzothiazine-2,4(3H)-dione

Employing the procedure of Example 1, parts A, B and C, with 3-butylpyrimidine-2,4(1H,3H)-dione and 2-aminobenzenethiol yielded the title compound.
m.p.: 250-251°C
IR:(KBr)cm⁻¹: 1717, 1650, 1518, 1428, 780

## Claims (Claims for the following Contracting State(s): AT, BE, CH, DE, DK, FR, GB, IT, LI, LU, NL, SE)

1. A compound of formula 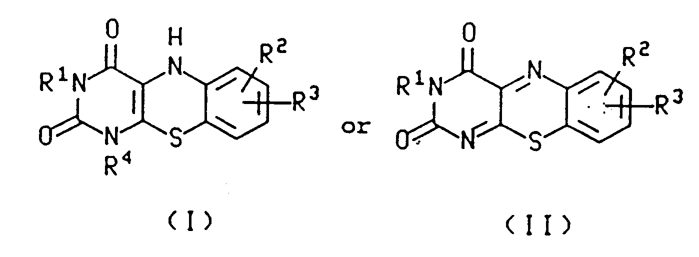 and the pharmaceutically-acceptable salts thereof, wherein R¹ is hydrogen or (C₁-C₆)alkyl; R² is hydrogen, halogen, (C₁-C₆)alkyl, (C₁-C₆)alkoxy or (C₁-C₆)halosubstituted alkyl; R³ is hydrogen or (C₁-C₆)alkyl; R⁴ is hydrogen, (C₁-C₆)alkyl, (C₃-C₈)cycloalkyl, (C₁-C₆)alkoxy-(C₁-C₆)alkyl or aryl-(C₁-C₆)alkyl; provided that in formula (I), R¹, R², R³ and R⁴ are not simultaneously hydrogen, R², R³ and R⁴ are not simultaneously hydrogen when R¹ is methyl and R² and R³ are not simultaneously hydrogen when R¹ and R⁴ are respectively methyl; provided further that in formula (II), R¹, R² and R³ are not simultaneously hydrogen and R² and R³ are not simultaneously hydrogen when R¹ is methyl; and provided further still that the groups R² and R³ may be attached to any available position on the ring in formula (I) or (II).

2. A compound according to claim 1 of the formula 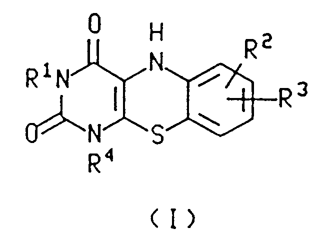 or a pharmaceutically-acceptable salt thereof wherein R¹, R², R³ and R⁴ are as defined in claim 1.

3. A compound according to claim 1 of the formula 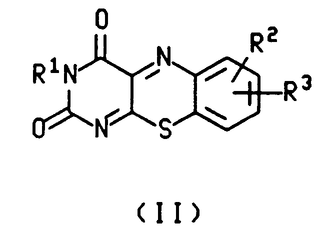 or a pharmaceutically-acceptable salt thereof wherein R¹, R², R³ and R⁴ are as defined in claim 1.

4. A compound or a pharmaceutically-acceptable salt thereof according to claim 2 wherein R¹ is (C₁-C₆)alkyl.

5. A compound or a pharmaceutically-acceptable salt thereof according to claim 4 wherein R¹ is methyl.

6. A compound or a pharmaceutically-acceptable salt thereof according to claim 5 wherein R² is halogen.

7. A compound or a pharmaceutically-acceptable salt thereof according to claim 5 wherein R² is (C₁-C₆)alkyl.

8. A compound or a pharmaceutically-acceptable salt thereof according to claim 5 wherein R² is (C₁-C₆)alkoxy.

9. A compound or a pharmaceutically-acceptable salt thereof according to claim 3 wherein R¹ is (C₁-C₆)alkyl.

10. A compound or a pharmaceutically-acceptable salt thereof according to claim 9 wherein R¹ is methyl.

11. A compound or a pharmaceutically-acceptable salt thereof according to claim 10 wherein R² is halogen.

12. A compound or a pharmaceutically-acceptable salt thereof according to claim 10 wherein R² is (C₁-C₆)alkyl.

13. A compound or a pharmaceutically-acceptable salt thereof according to claim 10 wherein R² is (C₁-C₆)alkoxy.

14. A compound or a pharmaceutically-acceptable salt thereof according to claim 10 wherein R² is (C₁-C₆)halo-substituted alkyl.

15. A pharmaceutical composition for the treatment of an allergic condition, inflammatory condition or cardiovascular disease in a mammal which comprises an effective amount of a compound according to claim 1 or a pharmaceutically-acceptable salt thereof and a pharmaceutically-acceptable diluent or carrier.

16. The use of a compound according to C1 for preparing a medicament for treating an allergic condition in a mammal which comprises administering to said mammal an effective amount of a compound according to claim 1 or a pharmaceutically-acceptable salt thereof.

17. The use of a compound according to C1 for preparing a medicament for treating an inflammatory condition in a mammal which comprises administering to said mammal an effective amount of a compound according to claim 1 or a pharmaceutically-acceptable salts thereof.

18. The use of a compound according to C1 for preparing a medicament for treating a cardiovascular disease in a mammal which comprises administering to said mammal an effective amount of a compound according to claim 1 or a pharmaceutically-acceptable salt thereof.

19. A compound according to claim 1, or a pharmaceutically acceptable salt thereof, or a pharmaceutical composition according to claim 15, for use as a medicament.

## Claims (Claims for the following Contracting State(s): ES, GR)

1. A process for preparing a compound of the formula 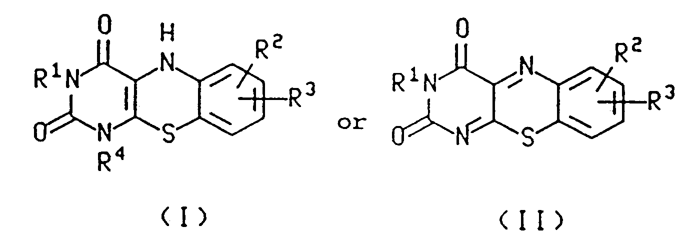 and the pharmaceutically-acceptable salts thereof, wherein R¹ is hydrogen or (C₁-C₆)alkyl; R² is hydrogen, halogen, (C₁-C₆)alkyl, (C₁-C₆)alkoxy or (C₁-C₆)halosubstituted alkyl; R³ is hydrogen or (C₁-C₆)alkyl; R⁴ is hydrogen, (C₁-C₆)alkyl, (C₃-C₈)cycloalkyl, (C₁-C₆)alkoxy-(C₁-C₆)alkyl or aryl-(C₁-C₆)alkyl; provided that in formula (I), R¹, R², R³ and R⁴ are not simultaneously hydrogen, R², R³ and R⁴ are not simultaneously hydrogen when R¹ is methyl and R² and R³ are not simultaneously hydrogen when R¹ and R⁴ are respectively methyl; provided further that in formula (II), R¹, R² and R³ are not simultaneously hydrogen and R² and R³ are not simultaneously hydrogen when R¹ is methyl; and provided further still that the groups R² and R³ may be attached to any available position on the ring in formula (I) or (II), which comprises reacting a compound of the formula 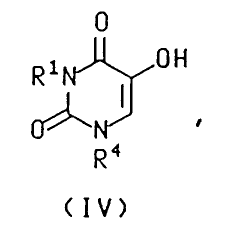 wherein R¹ and R⁴ are as defined above, with N-bromosuccinimide and reacting the product thereof with a compound of the formula 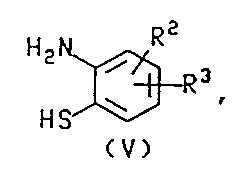 wherein R² and R³ are as defined above, at reflux to yield a compound of formula (I); and when a compound of formula (II) is desired, reacting a corresponding compound of formula (I) wherein R⁴ is hydrogen with an oxidant to yield a compound of formula (II); and when a pharmaceutically-acceptable salt of a compound of formula (I) or formula (II) is desired, converting the compound of formula (I) or formula (II) to the pharmaceutically-acceptable salt thereof by methods known per se.

2. A process according to claim 1 wherein a compound of formula (I) or a pharmaceutically-acceptable salt is prepared.

3. A process according to claim 1 wherein a compound of formula (II) or a pharmaceutically-acceptable salt thereof is prepared.

## Patentansprüche (Patentansprüche für folgende(n) Vertragsstaat(en): AT, BE, CH, DE, DK, FR, GB, IT, LI, LU, NL, SE)

1. Verbindung der Formel 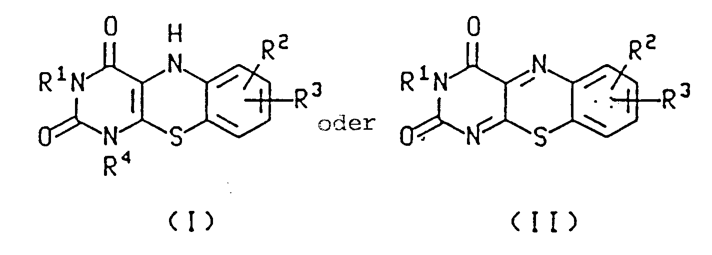 und die pharmazeutisch akzeptablen Salze derselben,
worin bedeuten:
R¹ Wasserstoff oder C₁-C₆-Alkyl,
R² Wasserstoff, Halogen, C₁-C₆-Alkyl, C₁-C₆-Alkoxy oder C₁-C₆-halogensubstituiertes Alkyl,
R³ Wasserstoff oder C₁-C₆-Alkyl,
R⁴ Wasserstoff, C₁-C₆-Alkyl, C₃-C₈-Cycloalkyl, C₁-C₆-Alkoxy-C₁-C₆-Alkyl oder Aryl-C₁-C₆-Alkyl,
vorausgesetzt, daß in der Formel (I) R¹, R², R³ und R⁴ nicht gleichzeitig für Wasserstoff stehen, R², R³ und R⁴ nicht gleichzeitig Wasserstoff bedeuten, wenn R¹ für Methyl steht, und R² und R³ nicht gleichzeitig Wasserstoff bedeuten, wenn R¹ und R⁴ jeweils Methyl bedeuten, des weiteren vorausgesetzt, daß in Formel (II) R¹, R² und R³ nicht gleichzeitig Wasserstoff bedeuten und R² und R³ nicht gleichzeitig für Wasserstoff stehen, wenn R¹ Methyl bedeutet, und des weiteren vorausgesetzt, daß die Gruppen R² und R³ in der Formel (I) oder (II) an irgendeiner beliebigen Position am Ring hängen können.

2. Verbindung nach Anspruch 1 der Formel 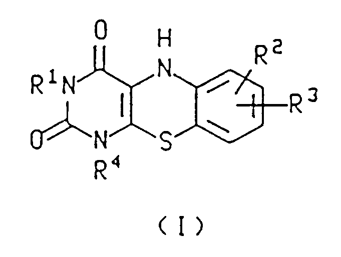 oder ein pharmazeutisch akzeptables Salz derselben, worin R¹, R², R³ und R⁴ die in Anspruch 1 angegebene Bedeutung besitzen.

3. Verbindung nach Anspruch 1 der Formel 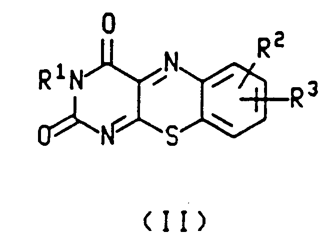 oder ein pharmazeutisch akzeptables Salz derselben, worin R¹, R², R³ und R⁴ die in Anspruch 1 angegebene Bedeutung besitzen.

4. Verbindung oder ein pharmazeutisch akzeptables Salz derselben nach Anspruch 2, wobei R¹ für C₁-C₆-Alkyl steht.

5. Verbindung oder ein pharmazeutisch akzeptables Salz derselben nach Anspruch 4, wobei R¹ für Methyl steht.

6. Verbindung oder ein pharmazeutisch akzeptables Salz derselben nach Anspruch 5, wobei R² für Halogen steht.

7. Verbindung oder ein pharmazeutisch akzeptables Salz derselben nach Anspruch 5, worin R² für C₁-C₆-Alkyl steht.

8. Verbindung oder ein pharmazeutisch akzeptables Salz derselben nach Anspruch 5, wobei R² für C₁-C₆-Alkoxy steht.

9. Verbindung oder ein pharmazeutisch akzeptables Salz derselben nach Anspruch 3, wobei R¹ für C₁-C₆-Alkyl steht.

10. Verbindung oder ein pharmazeutisch akzeptables Salz derselben nach Anspruch 9, wobei R¹ für Methyl steht.

11. Verbindung oder ein pharmazeutisch akzeptables Salz derselben nach Anspruch 10, wobei R² für Halogen steht.

12. Verbindung oder ein pharmazeutisch akzeptables Salz derselben nach Anspruch 10, wobei R² für C₁-C₆-Alkyl steht.

13. Verbindung oder ein pharmazeutisch akzeptables Salz derselben nach Anspruch 10, wobei R² für C₁-C₆-Alkoxy steht.

14. Verbindung oder ein pharmazeutisch akzeptables Salz derselben nach Anspruch 10, wobei R² für C₁-C₆-halogensubstituiertes Alkyl steht.

15. Pharmazeutische Zubereitung zur Behandlung eines allergischen Zustandes, eines Entzündungszustandes oder einer kardiovaskulären Erkrankung bei einem Säugetier, umfassend eine wirksame Menge einer Verbindung nach Anspruch 1 oder eines pharmazeutisch akzeptablen Salzes derselben und ein pharmazeutisch akzeptables Verdünnungsmittel oder einen pharmazeutisch akzeptablen Träger.

16. Verwendung einer Verbindung nach Anspruch 1 zur Herstellung eines Medikaments zur Behandlung eines allergischen Zustandes bei einem Säugetier, umfassend ein Verabreichen einer wirksamen Menge einer Verbindung nach Anspruch 1 oder eines pharmazeutisch akzeptablen Salzes derselben an ein Säugetier.

17. Verwendung einer Verbindung nach Anspruch 1 zur Herstellung eines Medikaments zur Behandlung eines Entzündungszustandes bei einem Säugetier, umfassend ein Verabreichen einer wirksamen Menge einer Verbindung nach Anspruch 1 oder eines pharmazeutisch akzeptablen Salzes derselben an ein Säugetier.

18. Verwendung einer Verbindung nach Anspruch 1 zur Herstellung eines Medikaments zur Behandlung einer kardiovaskulären Erkrankung bei einem Säugetier, umfassend eine Verabreichung einer wirksamen Menge einer Verbindung nach Anspruch 1 oder eines pharmazeutisch akzeptablen Salzes derselben an ein Säugetier.

19. Verbindung nach Anspruch 1 oder ein pharmazeutisch akzeptables Salz derselben oder eine pharmazeutische Zubereitung nach Anspruch 15 zur Verwendung als ein Medikament.

## Patentansprüche (Patentansprüche für folgende(n) Vertragsstaat(en): ES, GR)

1. Verfahren zur Herstellung einer Verbindung der Formel 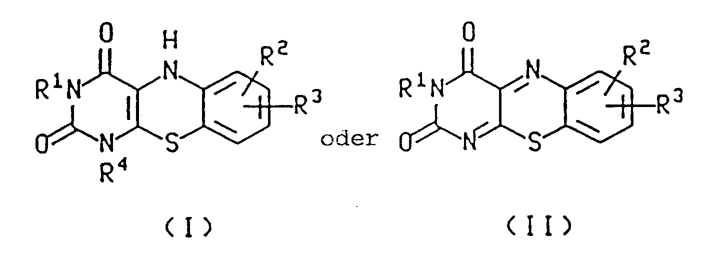 oder der pharmazeutisch akzeptablen Salze derselben,
worin bedeuten:
R¹ Wasserstoff oder C₁-C₆-Alkyl,
R² Wasserstoff, Halogen, C₁-C₆-Alkyl, C₁-C₆-Alkoxy oder C₁-C₆-halogensubstituiertes Alkyl,
R³ Wasserstoff oder C₁-C₆-Alkyl,
R⁴ Wasserstoff, C₁-C₆-Alkyl, C₃-C₈-Cycloalkyl, C₁-C₆-Alkoxy-C₁-C₆-Alkyl oder Aryl-C₁-C₆-Alkyl,
vorausgesetzt, daß in der Formel (I) R¹, R², R³ und R⁴ nicht gleichzeitig für Wasserstoff stehen, R², R³ und R⁴ nicht gleichzeitig Wasserstoff bedeuten, wenn R¹ für Methyl steht, und R² und R³ nicht gleichzeitig Wasserstoff bedeuten, wenn R¹ und R⁴ jeweils Methyl bedeuten, des weiteren vorausgesetzt, daß in Formel (II) R¹, R² und R³ nicht gleichzeitig Wasserstoff bedeuten und R² und R³ nicht gleichzeitig für Wasserstoff stehen, wenn R¹ Methyl bedeutet, und des weiteren vorausgesetzt, daß die Gruppen R² und R³ in der Formel (I) oder (II) an irgendeiner beliebigen Position am Ring hängen können, umfassend ein Umsetzen einer Verbindung der Formel 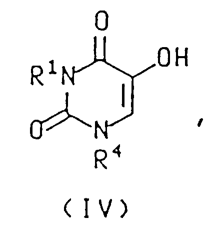 worin R¹ und R⁴ die oben angegebene Bedeutung besitzen, mit N-Bromsuccinimid und Umsetzung des Produkts derselben mit einer Verbindung der Formel 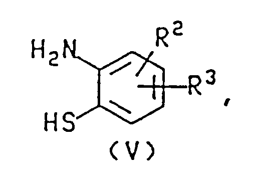 worin R² und R³ die oben angegebene Bedeutung besitzen bei Rückflußtemperatur unter Bildung einer Verbindung der Formel (I) und bei Anstreben einer Verbindung der Formel (II) Umsetzen einer entsprechenden Verbindung der Formel (I), worin R⁴ für Wasserstoff steht, mit einem Oxidationsmittel unter Bildung einer Verbindung der Formel (II) und bei Anstreben eines pharmazeutisch akzeptablen Salzes einer Verbindung der Formel (I) oder Formel (II) Umwandeln der Verbindung der Formel (I) oder Formel (II) in das pharmazeutisch akzeptable Salz derselben nach an sich bekannten Verfahren.

2. Verfahren nach Anspruch 1, wobei eine Verbindung der Formel (I) oder ein pharmazeutisch akzeptables Salz hergestellt wird.

3. Verfahren nach Anspruch 1, wobei eine Verbindung der Formel (II) oder ein pharmazeutisch akzeptables Salz derselben hergestellt wird.

## Revendications (Revendications pour l'(les) Etat(s) contractant(s) suivant(s): AT, BE, CH, DE, DK, FR, GB, IT, LI, LU, NL, SE)

1. Composé de formule : 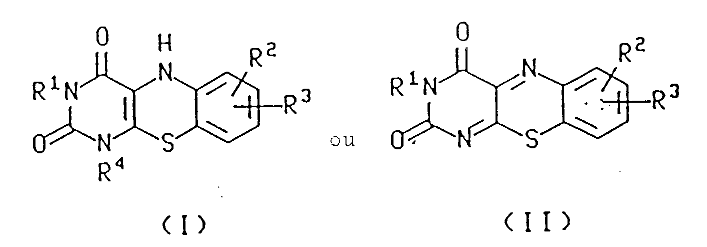 et ses sels pharmaceutiquement acceptables, formule dans laquelle R¹ représente l'hydrogène ou un groupe alkyle en C₁ à C₆ ; R² représente l'hydrogène, un halogène, un groupe alkyle en C₁ à C₆, alkoxy en C₁ à C₆ ou alkyle en C₁ à C₆ substitué avec un halogène ; R³ représente l'hydrogène ou un groupe alkyle en C₁ à C₆ ; R⁴ représente l'hydrogène, un groupe alkyle en C₁ à C₆, cycloalkyle en C₃ à C₈, (alkoxy en C₁ à C₆) -(alkyle en C₁ à C₆) ou aryl-(alkyle en C₁ à C₆) ; sous réserve que, dans la formule (I), R¹, R², R³ et R⁴ ne représentent pas simultanément l'hydrogène, R², R³ et R⁴ ne représentent pas simultanément l'hydrogène lorsque R¹ représente un groupe méthyle et R² et R³ ne représentent pas simultanément l'hydrogène lorsque R¹ et R⁴ représentent respectivement un groupe méthyle ; sous réserve en outre que, dans la formule (II), R¹, R² et R³ ne représentent pas simultanément l'hydrogène et R² et R³ ne représentent pas simultanément l'hydrogène lorsque R¹ représente un groupe méthyle ; et sous réserve en outre que les groupes R² et R³ puissent être fixés à n'importe quelle position disponible sur le noyau dans la formule (I) ou (II).

2. Composé suivant la revendication 1, de formule : 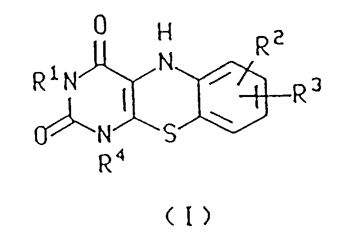 ou un de ses sels pharmaceutiquement acceptables, formule dans laquelle R¹, R², R³ et R⁴ répondent aux définitions suivant la revendication 1.

3. Composé suivant la revendication 1, de formule : 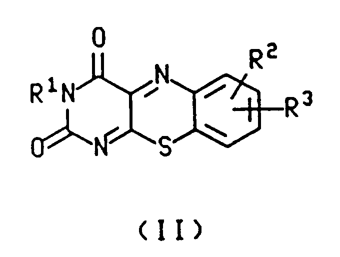 ou un de ses sels pharmaceutiquement acceptables, formule dans laquelle R¹, R², R³ et R⁴ répondent aux définitions suivant la revendication 1.

4. Composé ou un de ses sels pharmaceutiquement acceptables suivant la revendication 2, dans lequel R¹ représente un groupe alkyle en C₁ à C₆.

5. Composé ou un de ses sels pharmaceutiquement acceptables suivant la revendication 4, dans lequel R¹ représente un groupe méthyle.

6. Composé ou un de ses sels pharmaceutiquement acceptables suivant la revendication 5, dans lequel R² représente un halogène.

7. Composé ou un de ses sels pharmaceutiquement acceptables suivant la revendication 5, dans lequel R² représente un groupe alkyle en C₁ à C₆.

8. Composé ou un de ses sels pharmaceutiquement acceptables suivant la revendication 5, dans lequel R² représente un groupe alkoxy en C₁ à C₆.

9. Composé ou un de ses sels pharmaceutiquement acceptables suivant la revendication 3, dans lequel R¹ représente un groupe alkyle en C₁ à C₆.

10. Composé ou un de ses sels pharmaceutiquement acceptables suivant la revendication 9, dans lequel R¹ représente un groupe méthyle.

11. Composé ou un de ses sels pharmaceutiquement acceptables suivant la revendication 10, dans lequel R² représente un halogène.

12. Composé ou un de ses sels pharmaceutiquement acceptables suivant la revendication 10, dans lequel R² représente un groupe alkyle en C₁ à C₆.

13. Composé ou un de ses sels pharmaceutiquement acceptables suivant la revendication 10, dans lequel R² représente un groupe alkoxy en C₁ à C₆.

14. Composé ou un de ses sels pharmaceutiquement acceptables suivant la revendication 10, dans lequel R² représente un groupe alkyle en C₁ à C₆ substitué avec un halogène.

15. Composition pharmaceutique destinée au traitement d'une affection allergique, d'une affection inflammatoire ou d'une maladie cardiovasculaire chez un mammifère, qui comprend une quantité efficace d'un composé suivant la revendication 1 ou d'un de ses sels pharmaceutiquement acceptables et un diluant ou support pharmaceutiquement acceptable.

16. Utilisation d'un composé suivant la revendication 1 pour la préparation d'un médicament destiné au traitement d'une affection allergique chez un mammifère, qui comprend l'administration audit mammifère d'une quantité efficace d'un composé suivant la revendication 1 ou d'un de ses sels pharmaceutiquement acceptables.

17. Utilisation d'un composé suivant la revendication 1 pour la préparation d'un médicament destiné au traitement d'une affection inflammatoire chez un mammifère, qui comprend l'administration audit mammifère d'une quantité efficace d'un composé suivant la revendication 1 ou d'un de ses sels pharmaceutiquement acceptables.

18. Utilisation d'un composé suivant la revendication 1 pour la préparation d'un médicament destiné au traitement d'une maladie cardiovasculaire chez un mammifère, qui comprend l'administration audit mammifère d'une quantité efficace d'un composé suivant la revendication 1 ou d'un de ses sels pharmaceutiquement acceptables.

19. Composé suivant la revendication 1, ou un de ses sels pharmaceutiquement acceptables, ou bien une composition pharmaceutique suivant la revendication 15, destiné à être utilisé comme médicament.

## Revendications (Revendications pour l'(les) Etat(s) contractant(s) suivant(s): ES, GR)

1. Procédé de préparation d'un composé de formule : 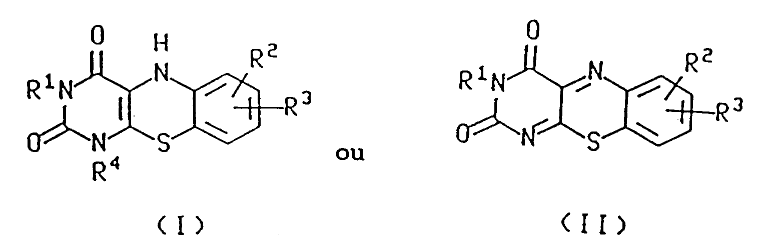 ou d'un de ses sels pharmaceutiquement acceptables, formule dans laquelle R¹ représente l'hydrogène ou un groupe alkyle en C₁ à C₆ ; R² représente l'hydrogène, un halogène, un groupe alkyle en C₁ à C₆, alkoxy en C₁ à C₆ ou alkyle en C₁ à C₆ substitué avec un halogène ; R³ représente l'hydrogène ou un groupe alkyle en C₁ à C₆ ; R⁴ représente l'hydrogène, un groupe alkyle en C₁ à C₆, cycloalkyle en C₃ à C₈, (alkoxy en C₁ à C₆) -(alkyle en C₁ à C₆) ou aryl-(alkyle en C₁ à C₆) ; sous réserve que, dans la formule (I), R¹, R², R³ et R⁴ ne représentent pas simultanément l'hydrogène, R², R³ et R⁴ ne représentent pas simultanément l'hydrogène lorsque R¹ représente un groupe méthyle et R² et R³ ne représentent pas simultanément l'hydrogène lorsque R¹ et R⁴ représentent respectivement un groupe méthyle ; sous réserve en outre que, dans la formule (II), R¹, R² et R³ ne représentent pas simultanément l'hydrogène et R² et R³ ne représentent pas simultanément l'hydrogène lorsque R¹ représente un groupe méthyle ; et sous réserve en outre que les groupes R² et R³ puissent être fixés à n'importe quelle position disponible sur le noyau dans la formule (I) ou (II), qui comprend la réaction d'un composé de formule : 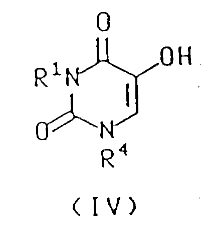 dans laquelle R¹ et R⁴ répondent aux définitions précitées, avec le N-bromosuccinimide, et la réaction du produit de cette réaction avec un composé de formule : 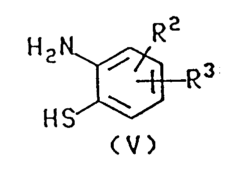 dans laquelle R² et R³ répondent aux définitions précitées, au reflux, ce qui donne un composé de formule (I) ; et, lorsqu'un composé de formule (II) est désiré, la réaction d'un composé correspondant de formule (I) dans laquelle R⁴ représente l'hydrogène avec un oxydant, ce qui donne un composé de formule (II) ; et, lorsqu'un sel pharmaceutiquement acceptable d'un composé de formule (I) ou de formule (II) est désiré, la transformation du composé de formule (I) ou de formule (II) en son sel pharmaceutiquement acceptable par des procédés en eux-mêmes connus.

2. Procédé suivant la revendication 1, dans lequel un composé de formule (I) ou un de ses sels pharmaceutiquement acceptables est préparé.

3. Procédé suivant la revendication 1, dans lequel un composé de formule (II) ou un de ses sels pharmaceutiquement acceptables est préparé.
